# EUROPEAN PATENT APPLICATION

(11) **EP 4 830 701 A1**
(43) Date of publication of application: **28.10.2026**
(21) Application number: 25196941.6
(22) Date of filing: 20.08.2025
(51) Int. Cl.: A61K 9/107, A61K 47/26, A61K 47/44, A61P 17/00

(54) **PROCESS FOR PRODUCTION OF POLYOL-FREE VEGETABLE OIL NANOEMULSION USING D-PHASE EMULSIFICATION, POLYOL-FREE VEGETABLE OIL NANOEMULSION, PHARMACEUTICAL COMPOSITION AND USE THEREOF**

(30) Priority: 22.04.2025 BR 102025007844
(71) Applicant: Alivira Saude Animal Ltda., 13098416 Sao Paulo/Campinas (BR); Universidade de São Paulo - USP, 05508-220 São Paulo (BR)
(72) Inventor: ARANTES, GERALDO JOSÉ, 38411-159 Uberlândia (BR); CARDOSO TOLEDO, LEANDRA, 38408-044 Uberlândia (BR); BOU CHACRA, NADIA ARACI, 04566-000 São Paulo (BR); FAGIONATO MASIERO, JÉSSICA, 05405-000 São Paulo (BR)
(74) Representative: Clarke, Modet y Cía., S.L.

(57) **Abstract**

The present invention refers to a process for producing a nanoemulsion comprising polyol-free vegetable oil, using the D-phase emulsification (DPE) method. Furthermore, the invention refers to the nanoemulsion comprising polyol-free vegetable oil, pharmaceutical composition and its use as a medicine in the treatment of atopy in mammals.

## Description

### FIELD OF THE APPLICATION

The present invention falls within the field of pharmacy and nanotechnology, and describes a process for obtaining a nanoemulsion preferably comprising polyol-free vegetable oil, in which the D-phase emulsification (DPE) technique is used. Furthermore, the present invention discloses a polyol-free vegetable oil nanoemulsion, a pharmaceutical composition and the use of the pharmaceutical composition in the treatment of atopy in mammals.

### BACkGROUND OF THE INVENTION AND STATE OF THE ART

Plant products, including fixed and volatile oils, may contain bioactive substances with the potential to be used in the treatment of different diseases (FAGIONATO MASIERO et al., 2021). Borage oil, derived from the seeds of the plant *Borago officinalis,* native to the Mediterranean region, contains high levels of essential fatty acids (EFAs), more specifically, omega-6, important for the structure and function of the skin. They play a fundamental role in the fluidity and flexibility of cell membranes and affect the function of membrane-associated proteins, such as receptors and enzymes (BACCOUCH et al., 2023). For this reason, this oil can be used as an adjuvant in the treatment of skin diseases, such as atopic dermatitis.

Atopic dermatitis is associated with abnormalities in the metabolism of (EFAs), particularly in the production of gamma-linolenic acid (GLA). This characteristic impairs the incorporation of EFAs into the phospholipid membrane (BALIĆ et al., 2020). EFAs are considered key components in membrane systems. Furthermore, they are metabolized into eicosanoids, specifically prostaglandins and leukotrienes, highly active and modulators of inflammatory, immune and proliferative responses, including epidermal cells (DENNIS; NORRIS, 2015). Therefore, the use of omega-6 EFAs can reverse symptoms caused by atopic dermatitis, such as inflammation, dryness and itching. The bioavailability of EFAs can be increased through the use of nanostructured system containing borage oil.

Nanoemulsions are oil-in-water (O/W) or water-in-oil (W/O) emulsions that are thermodynamically unstable (GUTIERREZ et al., 2008; SOLANS; SOLE, 2012), with an average particle size of approximately 100 to 1000 nm. Nanoemulsions remain resistant to sedimentation or creaming due to their reduced particle size, and ionic and non-ionic surfactants are often used in O/W nanoemulsions to stabilize them against flocculation, aggregation and agglomeration, through steric and electrostatic effects (ANTON; BENOIT; SAULNIER, 2008).

The "Ostwald ripening" phenomenon is recognized as the main source of destabilization in O/W nanoemulsions, caused by the diffusion of smaller particles towards larger ones, due to the Laplace pressure discrepancy (SOLANS; SOLÉ, 2012; TAYLOR, 1998). As it is a thermodynamically unstable system, the formation of the nanoemulsion does not occur spontaneously, requiring the application of energy, mechanical or chemical, for its formation. As a result, obtaining a nanoemulsion, unlike a microemulsion, is strongly conditioned by the process used to reduce the particles to nanometric size (SOLANS et al., 2005).

In the context of obtaining nanoemulsions, this process can be divided into two main mechanisms: the mechanical, high-energy process, and the physicochemical, low-energy process. Although the former has been the most frequently studied over the past decades-employing techniques such as high-pressure homogenization, microfluidization, sonication, and high-amplitude ultrasound, there is growing interest in low-energy processes due to their favorable cost-benefit ratio, as they do not require specific equipment for nanoemulsion production.

Low-energy processes are considered efficient because they reduce the need for energy costs, especially in industrial-scale production. In the physicochemical method, the most notable techniques include Phase Inversion Temperature (PIT), Phase Inversion Composition (PIC), Spontaneous Emulsification (SE) (ANTON; BENOIT; SAULNIER, 2008), and, more
recently, D-Phase Emulsification (DPE). High-pressure homogenization, as a high-energy method, and D-phase emulsification, as a low-energy method, were the methods selected to obtain borage oil nanoemulsions of the present invention because they present specific advantages when compared to other processes.

Conventional (low energy) physicochemical processes, such as PIT, PIC and Spontaneous Emulsification, require precise adjustment of the hydrophilic-lipophilic balance (HLB) of the system, the temperature and/or the addition method of the different phases to obtain nanoemulsions. The presence of and transition through the microemulsion (ME) or liquid crystal (LC) phase are required in these processes. This implies the need to invest time in adjusting the HLB, in addition to requiring a high concentration of surfactant to form LC or ME. This characteristic makes these conventional methods of obtaining low-energy nanoemulsions limited and with potentially high toxicity, due to the need for a high concentration of surfactant in the formulation (ANTON; BENOIT; SAULNIER, 2008). Surfactants have intrinsic toxicity and should be used in the lowest possible concentration. Furthermore, the rigid structure of the CL often results in phase separation with high oil concentrations, for example 20-30% (SAGITANI, 1988).

D-phase emulsification (DPE), presented in the 1980s by Sagitani et al., offers the advantage of obtaining fine and uniform emulsions without the need for expensive equipment and with a reduced concentration of surfactants/stabilizers. The DPE process offers advantages when compared to conventional phase inversion methods, including the formation of fine emulsions with low surfactant concentration, not requiring strict adjustment of the HLB when compared to PIC, PIT and SE. An additional advantage of DPE refers to the possibility of forming a nanoemulsion with a single hydrophilic surfactant (considered safer when compared to lipophilic surfactants) and obtaining highly stable emulsions at high temperatures, differently from the PIT process (SAGITANI; NABETA; NAGAI, 1991). Furthermore, unlike spontaneous emulsification methods, the DPE process does not require the presence of an organic solvent. This characteristic facilitates its use on an industrial scale.

Furthermore, the DPE process offers advantages when compared to conventional phase inversion methods, including the formation of fine emulsions with low surfactant concentration, not requiring strict adjustment of the HLB when compared to PIC, PIT and SE. An additional advantage of DPE refers to the possibility of forming a nanoemulsion with a single hydrophilic surfactant (considered safer when compared to lipophilic surfactants) and obtaining highly stable emulsions at high temperatures, differently from the PIT process (SAGITANI; NABETA; NAGAI, 1991). Furthermore, unlike spontaneous emulsification methods, the DPE process does not require the presence of an organic solvent. This characteristic facilitates its use on an industrial scale.

Unlike conventional low-energy methods, in the DPE process, the use of polyol as a surfactant is considered an essential component of the system, modifying the association properties of non-ionic surfactants with the medium (MURAKAMI et al., 2005; SAGITANI, 1986). Diols such as 1,3-
butylene glycol increase the surfactant HLB, directing it to the aqueous phase (formation of mixed micelles with non-ionic surfactants). Glycerol, D-sorbitol and polyethylene glycols reduce the HLB of the surfactant by directing it to the lipophilic phase, reducing the affinity between the non-ionic surfactant and water (SAGITANI; IKEDA; OGO, 1984).

By applying the DPE process, ENDOO; SAGITANI, (1991) successfully obtained an O/W nanoemulsion containing 20.0% (m/m) of olive oil and a particle size of 500 nm, using a hydrophilic surfactant (ethoxylated oleyl alcohol) at a concentration of 2.0% (m/m), and a temperature of 60
°C. It is important to highlight that this achievement was made without the need for any specialized devices, such as high-pressure or shear equipment. However, it is worth noting that, despite the success of this study, the application of the D-phase emulsification (DPE) process has been underutilized in recent decades, and to date, no evidence has been found of advances in the development of nanoemulsions containing more than 20.0% (w/w) borage oil with an average hydrodynamic diameter (AHD) below 500 nm.

The use of statistical tools, such as design of experiments (DoE), is crucial for the development and optimization of nanoemulsions. DoE allows for a more comprehensive assessment of the effects of process and formulation variables on quality attributes, resulting in more efficient optimization with fewer experiments. This quality-by-design approach is essential to ensure the consistency and efficacy of nanoemulsions, facilitating their clinical application and commercialization.

Design of experiments (DoE) is an important statistical tool in the research and development of nanostructured systems, in the quality by design approach. DoE allows in-depth knowledge about the influence of formula components and process parameters on the critical quality attributes of the preparation. This tool allows the variation of different factors at the same time. As a consequence, this tool allows efficient optimization using a reduced number of experiments (GUTIERREZ et al., 2008).

The use of a statistical tool in the preparation of nanoemulsion by DPE aimed to optimize it, containing a high concentration of borage oil (greater than or equal to 25.0% m/m) and a hydrophilic surfactant containing a low concentration (less than or equal to 4.0% m/m). Furthermore, the tool allows disclosing the influence of independent variables (oil concentration, surfactant concentration, glycerin concentration and initial water concentration) on the response (particle size). The target quality profile of the system was: AHD (average hydrodynamic diameter) below 500 nm, PI (polydispersity index) below 0.3, and zeta potential lower than -20 mV.

US8337931 patent discloses compositions and methods
to prepare foods and drinks that contain additives. Additives include nutraceuticals, pharmaceuticals, and supplements, such as essential fatty acids, including omega-3 fatty acids, omega-6 fatty acids, conjugated fatty acids, and other fatty acids; phytochemicals, including phytosterols; other oils; and coenzymes, including Coenzyme Q10 and other oil-based additives.

Although patent US8337931 discloses vegetable oil, borage oil, and the particle size range is similar to the present invention, there are relevant differences between both inventions, such as: the method of the US patent is carried out by high shear and has additional steps that employ heating and cooling. Furthermore, the US patent teaches a stirring speed range between 850 and 1200 rpm, and a speed reduction to 25 or 50 rpm to reduce air incorporation into the nanoemulsion.

Moreover, patent US8337931 teaches a preparation order for the nanoemulsion that differs from the present invention, in which the surfactant is added first, followed by the preservative, then the solvent, subsequently the co-surfactant, the non-polar compound, and finally the emulsion stabilizer. The invention now proposed uses only borage oil, surfactant and water in the formulation, and the stirring speed range is limited to 300 rpm.

Patent document CN105963335 discloses a nanoscale borage oil microemulsion and a method of preparing the nanoscale borage oil microemulsion. Nanoscale borage oil microemulsion is prepared from the following components: 2.5 to 5 parts of borage oil, 3 to 6 parts of citric acid fatty acid glyceride, 3 to 7 parts of sorbitan monooleate, 2 to 5 parts of sodium coconut oil acyl hydroxyethyl sulfonate, 0.5 to 3 parts of isopropyl myristate, 3 to 6 parts of polyglyceryl-10 monostearate, 2 to 5 parts of N-acyl sarcosine oil, 2 to
4 parts of polyglyceryl fatty acid ester, 0.5 to 2 parts of sucrose acetate isobutyrate, 6 to 10 parts of glycerin, 2 to 5 parts of polyethylene glycol 400, and 100 to 140 parts of water. The disclosed preparation method comprises the following steps: heating, mechanical stirring, uniform mixing, and high-shear processing. The nanoscale borage oil microemulsion prepared and obtained by patent document CN105963335 has the advantages of: small average grain diameter and strong dissolution property. Furthermore, according to a Chinese patent document, the preparation method has characteristics such as simple steps and ease of industrial production.

However, patent document CN105963335, unlike the invention proposed herein, discloses a method of manufacturing microemulsion using high-speed shearing between 5000 and 7000 rpm, which is considered a high-energy method. Moreover, the method described in the Chinese patent document includes steps that are not present in the current invention: a heating step and a microemulsion formation step with a stirring speed between 50 and 150 rpm.

In contrast, the present invention discloses a minimalist formulation containing only three components - borage oil, surfactant, and water - which showed remarkable results in physicochemical essays. This stands in stark contrast to Chinese patent document CN105963335, which describes a microemulsion using borage oil, as in the current invention, but includes additional components such as citric acid fatty acid glyceride, sorbitan monooleate, sodium cocoyl isethionate, isopropyl myristate, decaglyceryl monostearate, N-oleoyl sarcosine, glycerol, propylene glycol, and water.

Patent US5690947 discloses compositions containing hydroxy acids and/or retinoids and also containing borage seed oil as an anti-irritant. The US patent describes that borage seed oil was found to be exceptionally effective (when compared to gamma-linolenic acid, or other anti-irritants containing gamma-linolenic acid or other known anti-irritants) in reducing irritation caused by hydroxy acids and/or retinoids. Although the US patent uses borage oil to prepare an emulsion, there are significant distinctions between this patent document and the present invention.

First of all, the invention proposed herein is not an emulsion, but rather a nanoemulsion for oral use, therefore, with a particle size much smaller than the particle size of patent US5690947, which is intended for topical administration. Furthermore, in the invention described herein, borage oil is used in high concentration, 50% m/m, while in patent US5690947 it is low, between 0.05 and 10% m/m.

As observed, the state of the art would benefit from a solution that presents a nanoemulsion production process containing a high concentration of borage oil greater than 20 to 50% m/m, preferably 50%, without the need for rigorous HLB adjustment and high-cost equipment, maintaining the surfactant with the lowest possible concentration (maximum 4.0% m/m), and at the same time achieving the necessary physical stability.

### BRIEF DESCRIPTION OF THE INVENTION

The present invention refers to a process for producing a stable vegetable oil nanoemulsion with a high oil concentration, greater than 20 to 50%, preferably 50.0% (w/w), without the use of polyol, average hydrodynamic diameter (AHD) between 200 and 400 nm, obtained by employing 3 to 5% w/w, preferably 4.0% (w/w) of a single hydrophilic surfactant, by means of the DPE process. These characteristics represent a ratio of 1:12 and 1:16 (surfactant: oil), therefore with a low concentration of surfactant. And the vegetable oil is such as avocado oil (*Persea americana*), sweet almond oil (*Prunus dulcis*), andiroba oil (*Carapa guianensis*), argan oil (*Argania spinosa*), hazelnut oil (*Corylus avellana*), babassu oil (*Orbignya phalerata*), baobab oil (*Adansonia digitata*), buriti oil (*Mauritia flexuosa*), camellia oil (*Camellia oleifera*), canola oil (*Brassica napus*), Brazil nut oil (*Bertholletia excelsa*), Maranhão nut oil (*Sterculia striata*), safflower oil (*Carthamus tinctorius*), chia oil (*Salvia hispanica*)*,* coconut oil (*Cocos nucifera*), copaiba oil (*Copaifera spp*.), crambe oil (*Crambe abyssinica*), apricot oil (*Prunus armeniaca*), prickly pear oil (*Opuntia ficus-indica*), rice bran oil (*Oryza sativa*), raspberry oil (*Rubus idaeus*), sunflower oil (*Helianthus annuus*), sesame oil (*Sesamum indicum*), guarana oil (*Paullinia cupana*), shea oil (*Vitellaria paradoxa*), lavender oil (*Lavandula angustifolia*), linseed oil (*Linum usitatissimum*), macadamia oil (*Macadamia integrifolia*), castor oil (*Ricinus communis*), passion fruit oil (*Passiflora edulis*)*,* marula oil (*Sclerocarya birrea*), tea tree oil (*Melaleuca alternifolia*), corn oil (*Zea mays*), moringa oil (*Moringa oleifera*), mustard oil (*Brassica juncea*), neem oil (*Azadirachta indica*), pecan oil (*Carya illinoinensis*), olive oil (*Olea europaea*), pequi oil (*Caryocar brasiliense*), patauá oil (*Oenocarpus bataua*), pracaxi oil (*Pentaclethra macroloba*), rosehip oil (*Rosa rubiginosa*), pumpkin seed oil (*Cucurbita pepo*), cottonseed oil (*Gossypium hirsutum*), grape seed oil (*Vitis vinifera*), tamanu oil (*Calophyllum inophyllum*), tucuma oil (*Astrocaryum vulgare*), cashew oil (*Anacardium occidentale*), cupuagu oil (*Theobroma grandiflorum*), cocoa oil (*Theobroma cacao*), emburana oil (*Amburana cearensis*), peanut oil (*Arachis hypogaea*), black currant oil (*Ribes nigrum*), seaweed oil (*Schizochytrium spp*.), krill oil (*Euphausia superba*), borage oil (*Borago officinalis*), fennel oil (*Foeniculum vulgare*), rosemary oil (*Rosmarinus officinalis*), milk thistle oil (*Silybum marianum*), prickly pear oil (*Opuntia ficus-indica*), annatto oil (*Bixa orellana*), chamomile oil (*Matricaria recutita*), peppermint oil (*Mentha piperita*), ginger oil (*Zingiber officinale*).

Furthermore, the present invention discloses a vegetable oil nanoemulsion, which is a platform for encapsulation of drugs with low water solubility selected from the group of immunosuppressants such as: azathioprine, cyclosporine, tacrolimus, sirolimus (rapamycin), everolimus, temsirolimus, zotarolimus, leflunomide, mycophenolate mofetil, mycophenolate sodium, gusperimus, pimecrolimus, fingolimod, baricitinib, ruxolitinib, tofacitinib, abatacept, belatacept, voclosporin and laquinimod; non-steroidal anti-inflammatory drugs such as: aceclofenac, mefenamic acid, niflumic acid, tiaprofenic acid, amtolmetin guacil, benoxaprofen, bromfenac, carprofen, celecoxib, ketoprofen, clonixin, dexibuprofen, dexketoprofen, diclofenac, diflunisal, etodolac, etofenamate, phenylbutazone, flurbiprofen, ibuprofen, indomethacin, ketorolac, lornoxicam, lumiracoxib, meclofenamate, meloxicam, nabumetone, naproxen, nimesulide, oxaprozin, parecoxib, piroxicam, proglumetacin, rofecoxib, sulindac, tenoxicam, valdecoxib, tolmetin, azapropazone and feprazone; steroidal anti-inflammatory drugs such as: dexamethasone, betamethasone, budesonide, clobetasol, diflorasone, fluocinolone acetonide, fluocortolone, fluticasone, halcinonide, hydrocortisone, methylprednisolone, mometasone, paramethasone, prednisolone, prednisone, triamcinolone, desoximetasone, cyclohexanolone, beclomethasone and alclomethasone; antihistamines such as: astemizole, bilastine, ebastine, loratadine, desloratadine, mizolastine, terfenadine, clemastine, chlorpheniramine, dexchlorpheniramine, brompheniramine, diphenhydramine, promethazine, doxylamine, cyproheptadine, meclizine, hydroxyzine, triprolidine, tripelennamine, dimetindene, oxatomide, azelastine, buclizine, chlorcyclizine, ketotifen, alimemazine, antazoline, trimeprazine, pheniramine and dexbrompheniramine; and antibiotics such as: azithromycin, clarithromycin, erythromycin base, telithromycin, clindamycin, lincomycin, rifampicin, rifabutin, rifapentine, isoniazid, ethionamide, para-aminosalicylic acid, dapsone, chloramphenicol, tiamulin, valnemulin, gramicidin, bacitracin, polymyxin B, colistin, griseofulvin, sodium fusidate, streptomycin, amikacin, capreomycin, spectinomycin, cefradine, cefaclor, cefixime, cefdinir, cefpodoxime, ceftibutene, minocycline, doxycycline, tetracycline, tigecycline, ciprofloxacin, levofloxacin, moxifloxacin, norfloxacin, ofloxacin, trovafloxacin, besifloxacin, gatifloxacin, gemifloxacin, sitafloxacin, prulifloxacin, finafloxacin, nadifloxacin and enoxacin.

This invention also discloses a pharmaceutical composition comprising the vegetable oil nanoemulsion as a platform preferably for cyclosporine at 1% (w/w).

The invention proposed herein also refers to the use of the pharmaceutical composition in the manufacture of a medicine for the treatment of atopy, hypercholesterolemia, premenstrual syndrome, insulin resistance, in supporting wound healing, diabetic neuropathy, metabolic syndrome, preferably atopic dermatitis in mammals, that is, humans or animals, selected from the group consisting of: dogs, cats, horses, cattle, sheep, goats, pigs, rabbits, hamsters, guinea pigs, rats, preferably dogs and cats.

An additional embodiment refers to the method of treating atopic dermatitis in mammals, i.e. humans or animals, preferably dogs and cats, using the pharmaceutical composition.

Finally, the invention also refers to the use of the pharmaceutical composition for use in the treatment of atopic dermatitis.

The use of the statistical tool employed in the present invention allowed the revelation of surprising, unpredictable phenomena of fundamental importance for the adequate obtaining of the nanoemulsion.

The low energy method, as used in the invention proposed herein, allows the development of nanoemulsions with low surfactant content, high oil concentration and without the use of polyol and organic solvents, that are surprising characteristics in the development of nanoemulsions. Furthermore, the low-energy method allows for scale-up, enabling industrial use.

### BRIEF DESCRIPTION OF THE FIGURES

The subject matter of the present invention will become completely clear in its technical aspects from the detailed description that will be made based on the figures listed below.
Figure 1A: illustrates the structure and visual appearance of the DPE process at a temperature of 25°C, showing the isotropic solution containing surfactant and initial water.
Figure 1B: illustrates the structure and visual appearance of the DPE process at a temperature of 25°C, showing the translucent/transparent viscous structure obtained after the addition of oil.
Figure 1C: illustrates the structure and visual appearance of the DPE process at a temperature of 25°C, showing the white and fluid O/W emulsion after the final addition of water.
Figure 2A: illustrates the visual appearance of the DPE process at a temperature of 25°C, showing an enlarged view of the viscous structure.
Figure 2B: illustrates the visual aspect of the DPE process at a temperature of 25°C on an enlarged scale of the O/W emulsion.
Figure 3A: Illustrates the response surface and contour plots of the AHD of the borage oil nanoemulsion, considering the variables: borage oil concentration, polysorbate 80, and initial water content. Response surface and contour plots represent the potential relationship between three variables by displaying the three-dimensional relationship between these variables in two dimensions. Therefore, Figure 3A shows the change in particle size (AHD) as different concentrations of borage oil and polysorbate 80 are used.
Figure 3B: illustrates the response surface and contour plots of the AHD of the borage oil nanoemulsion, considering the variables concentration of borage oil, polysorbate 80 and initial water. Response surface and contour plots represent the potential relationship between three variables by displaying the three-dimensional relationship between these variables in two dimensions. Therefore, Figure 3B shows the change in particle size (AHD) as different concentrations of borage oil and initial water are used.
Figure 3C: illustrates the response surface and contour plots of the AHD of the borage oil nanoemulsion, considering the variables concentration of borage oil, polysorbate 80 and initial water. Response surface and contour plots represent the potential relationship between three variables by displaying the three-dimensional relationship between these variables in two dimensions. Therefore, Figure 3C shows the change in particle size (AHD) as different concentrations of polysorbate 80 and initial water are used.
Figure 4: Illustrates the main effects plots for the AHD as a function of the components and preparation variables using the DPE process.
Figure 5A: illustrates the toxicity assay of the polyol-free borage oil nanoemulsion prepared by the DPE method in the invertebrate model Galleria mellonella, showing the survival rate (A) of *G. mellonella* larvae injected with: PBS: phosphate-buffered saline solution, pH 7.4; 25% borage oil nanoemulsion; 50% borage oil nanoemulsion is shown.
Figure 5B: Illustrates the toxicity assay of the polyol-free borage oil nanoemulsion prepared by the DPE method in the invertebrate model Galleria mellonella, showing the health index (B) of *G. mellonella* larvae injected with: PBS: sodium phosphate buffer solution, pH 7.4; 25% borage oil nanoemulsion; 50% borage oil nanoemulsion is demonstrated.

### DETAILED DESCRIPTION OF THE INVENTION

The present invention refers to a process for producing vegetable oil nanoemulsion by DPE based on the ENDOO method; SAGITANI, (1991), in which three components are used: water, a nonionic surfactant, such as polysorbate 20, polysorbate 40, polysorbate 60, polysorbate 80, sorbitan monolaurate, sorbitan monopalmitate, sorbitan monoleate, sorbitan trioleate, lecithin, glyceryl stearate, glyceryl laurate, glyceryl oleate, glyceryl cetearate, caprylyl/capryl glucoside, decyl glucoside, lauryl polyglycoside, glyceryl isostearate, cetyl alcohol ethoxylate, octoxynol-9, nonoxynol-9, poloxamer 188, poloxamer 407, poloxamine 904, polyethylene glycol 400, polyethylene glycol 600, polyethylene glycol 1000, polyethylene glycol 3350, PEG-8 dimethicone, PEG-12 dimethicone, lauryl methacrylate, cholesterol ethoxylate, methylglucamine, polyethylene glycol distearate, methyl glucose ester, methyl glucose phosphate, polyglyceryl-4 ester, polyglyceryl-10 laurate, glyceryl ricinoleate, preferably polysorbate 80, and the vegetable oil such as avocado oil (*Persea americana*), sweet almond oil (*Prunus dulcis*), andiroba oil (*Carapa guianensis*), argan oil (*Argania spinosa*), hazelnut oil (*Corylus avellana*), babassu oil (*Orbignya phalerata*), baobab oil (*Adansonia digitata*), buriti oil (*Mauritia flexuosa*), camellia oil (*Camellia oleifera*), canola oil (*Brassica napus*), Brazil nut oil (*Bertholletia excelsa*), Maranhão nut oil (*Sterculia striata*), safflower oil (*Carthamus tinctorius*), chia oil (*Salvia hispanica*), coconut oil (*Cocos nucifera*), copaiba oil (*Copaifera spp*.), crambe oil (*Crambe abyssinica*), apricot oil (*Prunus armeniaca*), prickly pear oil (*Opuntia ficus-indica*), rice bran oil (*Oryza sativa*), raspberry oil (*Rubus idaeus*), sunflower oil (*Helianthus annuus*), sesame oil (*Sesamum indicum*), guarana oil (*Paullinia cupana*), shea oil (*Vitellaria paradoxa*), lavender oil (*Lavandula angustifolia*), linseed oil (*Linum usitatissimum*), macadamia oil (*Macadamia integrifolia*), castor oil (*Ricinus communis*), passion fruit oil *(Passiflora edulis),* marula oil (*Sclerocarya birrea*), tea tree oil (*Melaleuca alternifolia*), corn oil (*Zea mays*), moringa oil (*Moringa oleifera*), mustard oil (*Brassica juncea*), neem oil (*Azadirachta indica*), pecan oil (*Carya illinoinensis*), olive oil (*Olea europaea*), pequi oil (*Caryocar brasiliense*), patauá oil (*Oenocarpus bataua*), pracaxi oil (*Pentaclethra macroloba*), rosehip oil (*Rosa rubiginosa*), pumpkin seed oil (*Cucurbita pepo*), cottonseed oil (*Gossypium hirsutum*), grape seed oil *(Vitis vinifera),* tamanu oil (*Calophyllum inophyllum*), tucuma oil (*Astrocaryum vulgare*), cashew oil (*Anacardium occidentale*), cupuaçu oil (*Theobroma grandiflorum*), cocoa oil (*Theobroma cacao*), emburana oil (*Amburana cearensis*), peanut oil (*Arachis hypogaea*), black currant oil (*Ribes nigrum*), seaweed oil (*Schizochytrium spp*.), krill oil (*Euphausia superba*), borage oil (*Borago officinalis*), fennel oil (*Foeniculum vulgare*), rosemary oil (*Rosmarinus officinalis*), milk thistle oil (*Silybum marianum*), prickly pear oil (*Opuntia ficus-indica),* annatto oil (*Bixa orellana*), chamomile oil (*Matricaria recutita*), peppermint oil (*Mentha piperita*), ginger oil (*Zingiber officinale*), the preparation temperature ranging from 10°C to 50°C, preferably 25°C.

In a second embodiment, this invention discloses a vegetable oil nanoemulsion, which is a platform for encapsulation of drugs with low water solubility selected from the group of immunosuppressants such as: azathioprine, cyclosporine, tacrolimus, sirolimus (rapamycin), everolimus, temsirolimus, zotarolimus, leflunomide, mycophenolate mofetil, mycophenolate sodium, gusperimus, pimecrolimus, fingolimod, baricitinib, ruxolitinib, tofacitinib, abatacept, belatacept, voclosporin and laquinimod; non-steroidal anti-inflammatory drugs such as: aceclofenac, mefenamic acid, niflumic acid, tiaprofenic acid, amtolmetin guacil, benoxaprofen, bromfenac, carprofen, celecoxib, ketoprofen, clonixin, dexibuprofen, dexketoprofen, diclofenac, diflunisal, etodolac, etofenamate, phenylbutazone, flurbiprofen, ibuprofen, indomethacin, ketorolac, lornoxicam, lumiracoxib, meclofenamate, meloxicam, nabumetone, naproxen, nimesulide, oxaprozin, parecoxib, piroxicam, proglumetacin, rofecoxib, sulindac, tenoxicam, valdecoxib, tolmetin, azapropazone and feprazone; steroidal anti-inflammatory drugs such as: dexamethasone, betamethasone, budesonide, clobetasol, diflorasone, fluocinolone acetonide, fluocortolone, fluticasone, halcinonide, hydrocortisone, methylprednisolone, mometasone, paramethasone, prednisolone, prednisone, triamcinolone, desoximetasone, cyclohexanolone, beclomethasone and alclomethasone; antihistamines such as: astemizole, bilastine, ebastine, loratadine, desloratadine, mizolastine, terfenadine, clemastine, chlorpheniramine, dexchlorpheniramine, brompheniramine, diphenhydramine, promethazine, doxylamine, cyproheptadine, meclizine, hydroxyzine, triprolidine, tripelennamine, dimetindene, oxatomide, azelastine, buclizine, chlorcyclizine, ketotifen, alimemazine, antazoline, trimeprazine, pheniramine and dexbrompheniramine; and antibiotics such as: azithromycin, clarithromycin, erythromycin base, telithromycin, clindamycin, lincomycin, rifampicin, rifabutin, rifapentine, isoniazid, ethionamide, para-aminosalicylic acid, dapsone, chloramphenicol, tiamulin, valnemulin, gramicidin, bacitracin, polymyxin B, colistin, griseofulvin, sodium fusidate, streptomycin, amikacin, capreomycin, spectinomycin, cefradine, cefaclor, cefixime, cefdinir, cefpodoxime, ceftibutene, minocycline, doxycycline, tetracycline, tigecycline, ciprofloxacin, levofloxacin, moxifloxacin, norfloxacin, ofloxacin, trovafloxacin, besifloxacin, gatifloxacin, gemifloxacin, sitafloxacin, prulifloxacin, finafloxacin, nadifloxacin and enoxacin.

In a third embodiment, the invention proposed herein refers to a pharmaceutical composition comprising the vegetable oil nanoemulsion and an immunosuppressant.

In a fourth embodiment, the present invention discloses the use of the pharmaceutical composition in the preparation of a medicament for the treatment of atopy, hypercholesterolemia, premenstrual syndrome, insulin resistance, in supporting wound healing, diabetic neuropathy, metabolic syndrome, preferably atopic dermatitis in mammals selected from the group of: dogs, cats, horses, cattle, sheep, goats, pigs, rabbits, hamsters, guinea pigs, rats, preferably dogs and cats.

### - Development of nanoemulsion using DPE process - Nanoemulsion Preparation

The process begins with the preparation of the aqueous phase comprising 0.5 to 1.5% initial water, 1 to 5% non-ionic surfactant, preferably polysorbate 80, preferably 4.0% (w/w), homogenized under stirring of 50 to 500 rpm, preferably 300 rpm, at a temperature of 10 to 50°C, preferably 25°C. The vegetable oil, preferably borage oil, was added by dripping in 5 to 50% of its total volume, preferably 50%, to this aqueous phase, with a controlled speed of 50 to 500 rpm, preferably 300 rpm using a magnetic or mechanical stirrer. After complete addition of oil, the system was kept under stirring for 10 to 30 minutes, preferably 20 minutes. The process was followed by the addition of 46.5 to 43.5% of the remaining water. After this last step, the oil-in-water (O/W) nanoemulsion remained under constant stirring at 50 to 500 rpm, preferably 300 rpm for 10 to 30 minutes, preferably 20 minutes.

### - Preparation of nanoemulsion on a enlarged scale

From the laboratory scale, a process was developed for its expansion, aiming to reach a quantity of 1 kg. The same components and parameters mentioned in "Nanoemulsion Preparation" were used. The changes made involved the type of stirrer, this time a mechanical stirrer (IKA, RW20 digital), and the prepared quantity, which was 1 kg. The borage oil was added slowly, but did not follow the dripping pattern of the previous model; the necessary volume of oil was added with the aid of a peristaltic pump (flow of 5 to 100 mL/minute, preferably 10 mL/minute), since the preparation volume was higher. As it is a physicochemical method, the water addition flow rate did not influence the quality characteristics of the nanoemulsion.

### - Box-Behnken experiment design

The influence of the nanoemulsion components on the AHD was analyzed using response surface methodologies, Box-Behnken (three factors at three levels: +1; 0; -1). A total of 15 preparations including 3 central points (formulas 5, 10 and 11) were performed (Table 2). Independent variables were borage oil (25.0; 37.5; 50.0% m/m), polysorbate 80 (2.0; 3.0; 4.0% m/m) and initial water (0.5; 1.0; 1.5% m/m). Dependent variable or response was the AHD of the nanoemulsion. The matrix was generated by the statistical software Minitab 17 (State College PA, USA).

**Table 1 - Box-Behnken experiment for evaluating the average hydrodynamic diameter and polydispersity index via polyol-free DPE process.**

| **Formulation** | **BO** | **T80** | **H2Oi** |
|---|---|---|---|
| 1 | 37,5 | 2,0 | 0,5 |
| 2 | 50,0 | 4,0 | 1,0 |
| 3 | 37,5 | 4,0 | 0,5 |
| 4 | 50,0 | 3,0 | 0,5 |
| 5 | 37,5 | 3,0 | 1,0 |
| 6 | 25,0 | 3,0 | 1,5 |
| 7 | 50,0 | 3,0 | 1,5 |
| 8 | 50,0 | 2,0 | 1,0 |
| 9 | 25,0 | 2,0 | 1,0 |
| 10 | 37,5 | 3,0 | 1,0 |
| 11 | 37,5 | 3,0 | 1,0 |
| 12 | 37,5 | 2,0 | 1,5 |
| 13 | 25,0 | 4,0 | 1,0 |
| 14 | 37,5 | 4,0 | 1,5 |
| 15 | 25,0 | 3,0 | 0,5 |

| | | | |
|---|---|---|---|
| BO: borage oil; T80: polysorbate 80; H2Oi: initial water | | | |

### - Average Hydrodynamic Diameter (AHD) and Polydispersity Index (PI)

The AHD and PI of the droplets were determined by dynamic light scattering, diluting the samples at a ratio of 1:100, in freshly prepared ultrapure water (Milli-Q^{®}), and observing them, in scattered light, at an angle of 90°. Particle size distribution and polydispersity index were determined using Zetasizer Nano ZS90 equipment (Malvern Instruments, Malvem, UK). Average diameters (based on the Stokes-Einstein equation) and PI were determined by three individual measurements. The polydispersity index (Equation 1) is defined as the standard **deviation of the particle size distribution (σ) divided by the average particle diameter (2a).** $IP = {\left(\frac{σ}{2 a}\right)}^{2}$ Equation 1

### - Zeta Potential (ZP)

The zeta potential was determined using Zetasizer ZS90 equipment (Malvern Instruments, Malvem, UK) by the electrophoretic mobility method, which is converted into zeta potential (ζ) (mV) by means of the Henry equation (Equation 2). Samples were diluted in Milli-Q^{®} ultra-pure water prior to measurement. ${U}_{E} = \frac{{2}_{E} ζ}{3 η} f \left(ka\right)$ Equation 2

Where η and ε are the viscosity and dielectric constant, respectively, of the solvent at a temperature of 25°C. UE, electrophoretic mobility and ζ, zeta potential. The applied field strength was 20 V/cm. The conditions for determining the zeta potential were determined using a conductivity versus zeta potential curve. The conductivity range evaluated was 50 µS/cm using 0.9 NaCl solution (m/V). The pH value was adjusted to 5.5 ± 0.2 by adding 0.01N HCl or 0.01N NaOH.

### - Nanoemulsion optimization

Minitab 17 statistical software (State College, PA, USA) was used to perform response optimization using the desire function approach. The tool allows determining the highest concentration of borage oil and the lowest concentration of surfactant in the formulation, which would allow its formation. Furthermore, a average hydrodynamic diameter (AHD) of less than 500 nm, preferably 350 nm, was sought. The desire function (d) was established on a scale from zero to 1, with 1 being the desire corresponding to 100%.

### - Verification of the mathematical model generated by the statistic analysis

Based on the optimization procedure, a new formulation was obtained. The observed and predicted AHD values for the nanoemulsions were determined, aiming to evaluate the adequacy of the mathematical model.

### - Physical stability

The evaluation of the nanoemulsion stability was conducted by analyzing the average hydrodynamic diameter (AHD), polydispersity index (PI) and zeta potential (ZP) and by visual observation of the formulations during the storage period at a temperature of 25 ± 0.5°C, for a period of twelve months. The preparations were kept in hermetically sealed borosilicate glass bottles in a climate chamber (Climacell MMM Group).

### - Toxicity in alternative model of Galleria mellonella

The toxicity essay was performed using larvae of *Galleria mellonella,* with a length between 2.0 and 2.5 cm and a weight ranging from 150 to 200 mg. The evaluated samples included borage oil (BO) and borage oil nanoemulsion prepared by the DPE method (BO = 25% w/w; BO = 50.0% w/w). A volume of 10 µL of the formulations was injected into the last false leg (from the English "proleg") of each larva (n=16 larvae per group). Sodium phosphate buffer at pH 7.4 was injected as a control for mechanical damage, and survival and morbidity of the groups were monitored daily for five days.

The following Examples provide the results of the polyol-free nanoemulsion preparation essay by the DPE method (Example 1), the expanded-scale polyol-free nanoemulsion preparation essay by the DPE method (Example 2), the Box-Benhken experimental design essay for the polyol-free formulation prepared by the DPE method (Example 3), the regression equation essay for the variables for the polyol-free formulation prepared by the DPE method (Example 4), the optimization essay for the polyol-free nanoemulsion and the verification of the mathematical model generated by the statistical analysis for the polyol-free formulation prepared by the DPE method (Example 5), the physical stability essay for the polyol-free formulation prepared by the DPE method (Example 6), the toxicity essay in an alternative model of *Galleria mellonella* for polyol-free formulation prepared by the DPE method (Example 7), essaying of polyol-free formulations optimized for physical stability (Example 8), and encapsulation essaying of the polyol-free borage oil formulation with cyclosporine in nanoemulsion by the DPE method (Example 9).

### EXAMPLE 1 - Preparation of polyol-free nanoemulsion for formulation prepared by DPE method

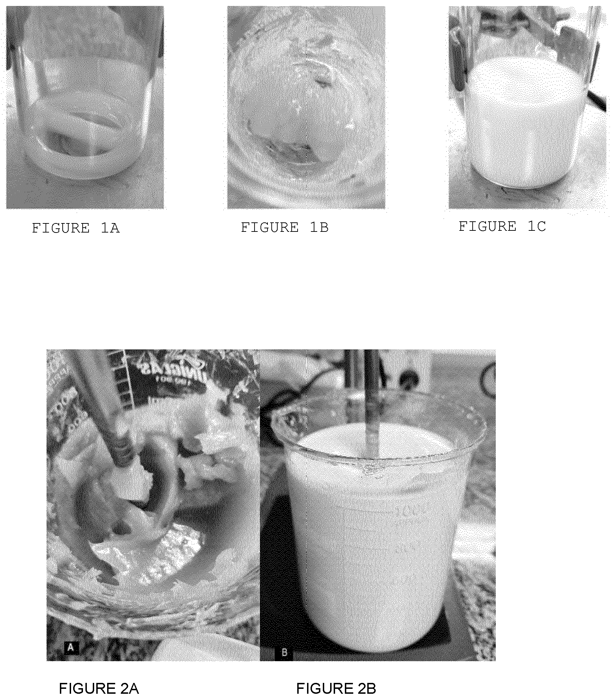

The isotropic solution comprising surfactant and aqueous phase (Figure 1A) demonstrated rapid dispersion of the oil phase upon its addition when subjected to stirring. The initial clear and isotropic solution gradually became cloudy as the oil concentration increased, resulting in an increase in viscosity. The system progressively evolved towards a translucent state, characterized by a high viscosity system (Figure 1B). Even in the presence of a high concentration of the oily phase, it was easily incorporated into this system, without the occurrence of phase separation. Then, by continuously adding the remaining portion of water, the fine oil particles of the internal phase, resulting from phase B, dispersed in the continuous aqueous phase, resulting in the formation of the final oil-in-water (O/W) nanoemulsion (Figure 1C).

This rapid dispersion of the oil phase in the isotropic solution is explained by the low interfacial tension between the added oil phase and the isotropic solution. The observed phenomena indicated the presence of an oil-in-surfactant (O/S) phase, in agreement with the procedure outlined by ENDOO; SAGITANI, (1991). Subsequently added water readily dispersed in the O/S phase, resulting in the formation of a final emulsion with a milky appearance. This phenomenon indicates that the external continuous phase did not consist of an oil phase (therefore, not characteristic of a water-in-oil emulsion, W/O, nor of a surfactant-in-oil emulsion, S/O). Briefly, during the continuous addition of the remaining water, the small oil droplets previously formed in the O/S phase dispersed in the aqueous phase, resulting in the formation of the oil-in-water (O/W) nanoemulsion. These characteristics are in accordance with the studies by SAGITANI, (1986), reinforcing the correct application of the DPE process in this essay, even though the polyol was excluded from this formulation.

### EXAMPLE 2 - Preparation of the polyol-free nanoemulsion on an enlarged scale for the formulation prepared using the DPE method

Following the method described in Example 1, the nanoemulsion was prepared on an enlarger scale to verify the possibility of transposing the method for industrial use. With the addition of oil, it was observed that the isotropic mixture, previously transparent, gradually became milky and greenish in color (Figure 2A). After complete addition of the borage oil, the viscous mass remained under stirring for 20 minutes and then the final water was slowly added. Then, by continuously adding the remaining portion of water, the fine oil particles of the internal phase, resulting from phase B, dispersed in the continuous aqueous phase, resulting in the formation of the final oil-in-water (O/W) nanoemulsion (Figure 7B). After preparation, the nanoemulsion presented AHD of 339.2 ± 3.8 nm, PI of 0.118 ± 0.090 and ZP of -35.7 ± 0.6 mV.

### EXAMPLE 3 - Box-Behnken design experiment for the polyol-free formulation prepared using the DPE method.

The experiments were conducted following a statistical approach, with 3 central points (preparations with the same composition: 5, 10 and 11) to increase the probability of detecting the significance of the input variables, estimating the variability (pure error) and statistically determining whether the relationship between the independent variables and the response is linear or not (MONTGOMERY, 2019). Furthermore, the experiments, a total of 15, were carried out randomly, without the need for new replications.

Response surface methodology (RSM) was applied to evaluate the influence and interactions of the following independent variables: borage oil concentration, surfactant concentration and initial water concentration, with AHD as the output response (dependent variable). The experiments resulted in formulations outside specifications (no emulsion formation), which did not allow subsequent statistical analysis of the data. However, these results guided decisions for further improvements in the process. Furthermore, they allowed us to identify the factors that resulted in the unpredictability of the formulation in this exploratory stage of the experimental design, in the DPE process. This step of the experimental design identified the optimized ranges of the independent variables and improved the understanding of the process, as described below, allowing the output response to remain within the specified range (CQA).

**Table 2 - First Box-Behnken experiment to evaluate the average hydrodynamic diameter and polydispersity index via polyol-free DPE process.**

| **Formulation** | **BO** | **T80** | **H2Oi** | **AHD** | **PI** | **ZP** |
|---|---|---|---|---|---|---|
| 1 | 37.5 | 2.0 | 0.5 | x | x | x |
| 2 | 50.0 | 4.0 | 1.0 | x | x | x |
| 3 | 37.5 | 4.0 | 0,5 | x | x | x |
| 4 | 50.0 | 3.0 | 0.5 | x | x | x |
| 5 | 37.5 | 3.0 | 1.0 | x | x | x |
| 6 | 25.0 | 3.0 | 1.5 | x | x | x |
| 7 | 50.0 | 3.0 | 1.5 | x | x | x |
| 8 | 50.0 | 2.0 | 1.0 | x | x | x |
| 9 | 25.0 | 2.0 | 1.0 | x | x | x |
| 10 | 37.5 | 3.0 | 1.0 | x | x | x |
| 11 | 37.5 | 3.0 | 1.0 | x | x | x |
| 12 | 37.5 | 2.0 | 1.5 | x | x | x |
| 13 | 25.0 | 4.0 | 1.0 | x | x | x |
| 14 | 37.5 | 4.0 | 1.5 | x | x | x |
| 15 | 25.0 | 3.0 | 0.5 | x | x | x |

| | | | | | | |
|---|---|---|---|---|---|---|
| BO: borage oil; T80: polysorbate 80; H2Oi: initial water; AHD: average hydrodynamic diameter; IP: polydispersity index; PZ: zeta potential. | | | | | | |

A new experimental design was generated using Minitab software and the results are presented in Table 7, with AHD ranging from 257.8 to 505.1 nm (IP values between 0.036 and 0.183), showing a narrow and monomodal distribution.

**Table 3 - Average hydrodynamic diameter and polydispersity index via polyol-free DPE process.**

| **Formulation** | **BO** | **T80** | **H2Oi** | **AH PI D** | | **ZP** |
|---|---|---|---|---|---|---|
| 1 | 25.0 | 4.0 | 1.00 | 264.3 ± 0.2 | 0.070 ± 0.025 | -23.0 ± 0.8 |
| 2 | 37.5 | 4.5 | 1.50 | 279.7 ± 3.7 | 0.065 ± 0.034 | -27.3 ± 1.1 |
| 3 | 37.5 | 4.5 | 1.00 | x | x | x |
| 4 | 37.5 | 4.0 | 1.25 | 312.6 ± 4.4 | 0.036 ± 0.025 | -26.9 ± 0.6 |
| 5 | 37.5 | 3.5 | 1.50 | 403.8 ± 3.3 | 0.174 ± 0.036 | -31.9 ± 0.9 |
| 6 | 50.0 | 4.0 | 1.00 | x | x | x |
| 7 | 50.0 | 4.5 | 1.25 | 505.1 ± 23.3 | 0.122 ± 0.104 | -27.7 ± 0.9 |
| 8 | 25.0 | 4.0 | 1.50 | 257.8 ± 3.9 | 0.141 ± 0.059 | -32.3 ± 1.2 |
| 9 | 37.5 | 3.5 | 1.00 | 453.8 ± 2.6 | 0.146 ± 0.035 | -28.4 ± 0.9 |
| 10 | 25.0 | 3.5 | 1.25 | 391.7 ± 4.6 | 0.163 ± 0.017 | -26.4 ± 0.3 |
| 11 | 37.5 | 4.0 | 1.25 | 339.9 ± 8.6 | 0.140 ± 0.021 | -28.2 ± 1.1 |
| 12 | 25,0 | 4,5 | 1,25 | 280,7 ± 4,3 | 0,050 ± 0,061 | -25,9 ± 0,4 |
| 13 | 50,0 | 4,0 | 1,50 | 447,1 ± 6,2 | 0,141 ± 0,066 | -27,0 ± 3,2 |
| 14 | 50,0 | 3,5 | 1,25 | 313,3 ± 0,8 | 0,183 ± 0,088 | -42,7 ± 1,4 |
| 15 | 37,5 | 4,0 | 1,25 | 355,8 ± 2,8 | 0,135 ± 0,043 | -28,2 ± 0,6 |

| | | | | | | |
|---|---|---|---|---|---|---|
| BO: borage oil; T80: polysorbate 80; H2Oi: initial water; AHD: average hydrodynamic diameter; IP: polydispersity index; ZP: zeta potential. | | | | | | |

**Table 4 - Analysis of variance of the different fitted response models and quadratic regression for the average hydrodynamic diameter of the nanoemulsion.**

| **Source** | **GL** | | **SQ (Aj.)** | **Q (Aj.) M** | **Value** F | **P-value** | **Total** |
|---|---|---|---|---|---|---|---|
| Model | | 7 | 459017 | 65574 | 38.64 | 0.000 | |
| Linear | | 3 | 312891 | 104297 | 61.46 | 0.000 | |
| BO | | 1 | 135408 | 135408 | 79.79 | 0.000 | **14** |
| T80 | | 1 | 42413 | 42413 | 24.99 | 0.002 | **470897** |
| H2Oi | | 1 | 135070 | 135070 | 79.59 | 0.000 | |
| Square | | 1 | 53883 | 53883 | 31.75 | 0.001 | |
| H2Oi*H2Oi | | 1 | 53883 | 53883 | 31.75 | 0.001 | |
| Interaction | | 3 | 92243 | 30748 | 18.12 | 0.001 | |
| BO*T80 | | 1 | 22952 | 22952 | 13.52 | 0.008 | |
| BO*H2Oi | | 1 | 13995 | 13995 | 8.25 | 0.024 | |
| T80*H2Oi | | 1 | 55296 | 55296 | 32.58 | 0.001 | |
| Error | | 7 | 11879 | 1697 | | | |
| Lack of adjustment | | 5 | 10932 | 2186 | 4.61 | 0.188 | |
| Pure error | | 2 | 948 | 474 | * | * | |

| **Model Summary** | | **R²** | | **R² (adj)** | | **R² (pred)** | |
|---|---|---|---|---|---|---|---|
| | | **97.48%** | | **94.95%** | | **84.17%** | |

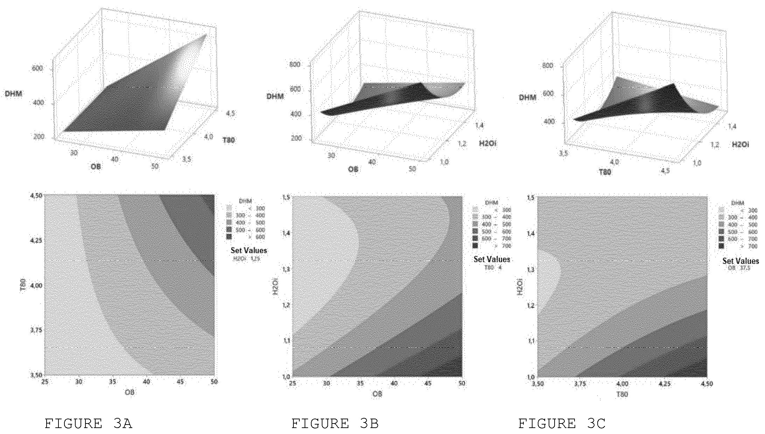

Analysis of variance (Table 4) disclosed that the independent variables presented statistical significance (p <0.05; □=0.05), as well as their interactions. The quadratic effect of initial water was also significant for the AHD of the nanoemulsion (p < 0.05; □=0.05). The lack of adjustment was not significant (p-value = 0.188), indicating that the proposed quadratic polynomial model is adequate. The effects described for AHD are illustrated in the response surface and contour plot (Figure 3).

From the plots in Figure 3, it can be observed that the AHD decreased as the initial water concentration increased. A quadratic effect was also observed between polysorbate 80 and initial water for AHD reduction.

### EXAMPLE 4 - Regression equation for the variables for polyol- free formulation prepared by the DPE method

The quadratic regression model demonstrated the coefficient of determination (R2) of 97.48% for the AHD, indicating that this response value can be attributed to the identified independent variables. R²adj, which reflects the correlation between experimental and predicted values, was 94.95%. This is a value close to R2, indicating a good statistical model. The predicted coefficient of determination (R²pred) was 84.17%, indicating the adequacy of the model in predicting responses to new observations. The regression equation of the variables in terms of uncoded factors is presented as follows: Equation 1: AHD (nm)= -735-14.4 BO+867 T80-853 H2Oi+1922 H2Oi*H2Oi +12.12 BO*T80-18.93 BO*H2Oi-941 T80*H2Oi

The equation describes the average particle size, considering linear, quadratic terms and interactions. Linear coefficients indicate the direct influence of each component on particle size. For example, the negative coefficient of -14.4 for BO suggests that increasing BO concentration is associated with decreasing particle size, while the positive coefficient (867) for T80 indicates that increasing the concentration of this component increases particle size. Furthermore, the presence of the quadratic term 1922 H2Oi*H2Oi discloses the nonlinear relationship between H2Oi concentration and particle size. Thus, by increasing the initial water concentration, there is an increase in particle size. In this case, the value of the coefficient (1922) has a surprising influence on the particle size. The interaction terms, such as +12.12 BO*T80 and -18.93 BO*H2Oi, indicate how combinations of different components influence particle size, either increasing or reducing it. Such phenomena cannot be predicted. For example, a positive coefficient of +12.12 for BO*T80 indicates that the joint increase in the concentrations of BO and T80 causes an increase in particle size. While the increase in BO and H2Oi concentrations reduces particle size. These phenomena were disclosed specifically for this formulation and for this method and cannot be predicted or inferred from the state of the art.

### EXAMPLE 5 - Optimization of polyol-free nanoemulsion and verification of the mathematical model generated by statistical analysis for polyol-free formulation prepared by DPE method

The optimized condition for nanoemulsion preparation, based on the regression equation, resulted in a nanoemulsion with a higher concentration of borage oil using a low concentration of surfactant. The optimum conditions within the desire function (1.0) were disclosed by combining borage oil concentration at 31.0% (m/m), surfactant concentration at 3.8% (m/m) and initial water concentration of 1.5% (m/m) (Figure 9). This resulted in a theoretical AHD of 350.0 nm. The experimental value resulting from these conditions was 300.5 ± 2.2 nm. The similar predicted and observed AHD value obtained from the optimized formula, as shown in Table 5, validated the proposed model.

**Table 5 - Theoretical and experimental values for the AHD of particles of the optimized formula.**

| **Practical AHD (nm)** | **Theoretical AHD (nm)** | **95% IC** | **IP** | **PZ (mV)** |
|---|---|---|---|---|
| 300.5 ± 2.2 | 350.0 | 284.6 **to** 415.4 | 0.25 ± 0.004 | -59.4 ± 3.8 |

In this invention, the use of the statistical approach disclosed the combination of optimized independent variables, surprisingly allowing to obtain nanoemulsion with AHD close to that of previous studies by the group of inventors of the invention now proposed (formulation containing glycerin, MASIERO, Jéssica Fagionato et al. Unveiling the optimal path: High-pressure homogenization and D-phase emulsification methods for borage oil nanoemulsion design. Industrial Crops and Products, v. 208, p. 117853, 2024.), maintaining satisfactory borage oil content (31.0% m/m) with low surfactant concentration (3.8% m/m). Similar to such studies, the influence of the initial water was statistically proven, as well as its interaction with the surfactant in the reduction of AHD and consequent formation of the nanoemulsion without the need for HLB adjustment.

### EXAMPLE 6 - Physical stability for a formulation free of polyol prepared by the DPE method

The physical stability of the nanoemulsion is a critical aspect in pharmaceutical development, being particularly challenging due to the Ostwald ripening phenomenon, which can occur over prolonged periods (KOROLEVA; NAGOVITSINA; YURTOV, 2018). This phenomenon refers to the progressive reduction of small droplets, while larger ones progressively increase during the storage period, eventually causing the complete dissolution of small droplets in the external phase (ROCCHIO et al., 2017). In the present invention, aiming to counteract this destabilizing effect, polysorbate 80 was selected as the preferred surfactant to promote stabilization.

For the DPE method, the AHD immediately after preparation was 328.9 ± 7.1 nm, and the final AHD after twelve months was 339.9 ± 5.2 nm, representing a variation of 3%. These results indicated that the preparation remained stable throughout the twelve-month evaluation period, with no indications of phase separation. The initial value of the zeta potential was -31.2 ± 3.5 mV immediately after preparation and -29.1 ± 3.1 mV after twelve months.

Briefly, the stability evaluations of the polyol-free borage oil nanoemulsion using the DPE method indicate its suitable potential for industrial use.

### EXAMPLE 7 - Toxicity in an alternative model of Gallery mellonella for polyol-free formulation prepared by DPE method

The toxicity essay was performed with larvae of *Galleria mellonella* measuring between 2.0 and 2.5 cm in length and weighing between 150 and 200 mg. The evaluated samples included pure borage oil, coarse borage oil emulsion, polyol-free borage oil nanoemulsion **prepared by the DPE method (BO = 50% w/w). A volume of 10 µL** of the formulations was injected into the last false leg of each larva (n = 16 larvae per group). A sodium phosphate buffer at pH 7.4 was injected as a mechanical damage control, and the survival and health index of the groups were monitored daily for five days.

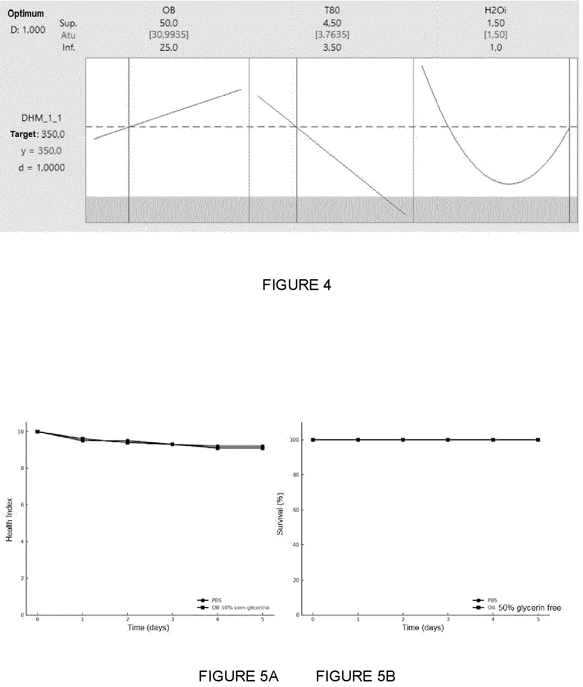

The toxicity of borage oil nanoemulsion by polyol-free DPE (50% w/w BO) was evaluated. The groups essayed with nanoemulsions prepared by the DPE method did not show significant toxicity (Figure 5A and Figure 5B), since the health index of the *Galleria mellonella* larvae was close to the maximum measured value, and the survival percentage for the essayed population was 100%. The health index is assessed according to a scoring system.

The assessment is made based on four categories: activity, cocoon formation, melanization and survival, assigning a specific score for each criterion. In the activity category, larvae that do not show movement receive 0 points. Those that demonstrate minimal movement only when stimulated receive 1 point. If the larva moves only when stimulated, but in a more evident way, the score given is 2 points. Larvae that move spontaneously, without the need for stimulus, receive the maximum score of 3 points.

Cocoon formation is also evaluated. Larvae that show no sign of cocoon formation receive 0 points. If the cocoon is in the process of forming, but incomplete, the score is 0.5 points. Finally, larvae that form a complete cocoon receive 1 point.

Melanization, an important indicator of immune response and possible infection, is scored as follows: completely black larvae receive 0 points, as this may indicate a high level of infection or necrosis. If the larva is black but has brown spots, it scores 1 point. When the larva is beige in color and has three or more dark spots, it receives 2 points. If the larva is beige and has less than three spots, the score given is 3 points. The best-case scenario, where there are no signs of melanization, receives the maximum score of 4 points. Finally, survival is assessed in a binary manner. Dead larvae score 0 points, while those that are alive score 2 points.

Based on this system, larvae considered healthy, that is, without infection or significant impairment, have a total score between 9 and 10 points (LOH et al., 2013).

### EXAMPLE 8 - Optimized polyol-free formulations in physical stability

With the aim of evaluating physical stability to achieve the longest possible shelf life and to facilitate scale-up, a formulation was prepared - in addition to the optimized one - with a higher oil concentration (Table 6).

**Table 6 - Formulations developed on laboratory and expanded scales, in stability assessment.**

| **Components (g)** | **Polyol Free** | | |
|---|---|---|---|
| | **Laboratory Scale (50 g)** | | **Enlarged Scale (1000 g)** |
| | **Ot.F.** | **Max.F.** | **Max.F.** |
| **BO** | 15.50 | 25.0 | 500.0 |
| **T80** | 1.88 | 2.0 | 40.0 |
| **H₂Oᵢ** | 0.75 | 1.0 | 20.0 |
| **H₂O_{f}** | 31. 87 | 22.0 | 440.0 |

| | | | |
|---|---|---|---|
| BO: borage oil; T80: polysorbate 80; H2Oi: initial water; H2Of: final water Ot.F.: optimized formulation; Max.F.: formulation with maximum concentration of borage oil. | | | |

These formulations remain in a climate chamber, at 30°C ± 2°C and 75% humidity for 12 months, being evaluated periodically (twelve months - laboratory scale, six months - expanded scale), they present AHD, PI and ZP in accordance with Table 7.

**Table 7 - AHD, PI and ZP of the stability formulations evaluated after a period of twelve months.**

| | ***Polyol free*** | | |
|---|---|---|---|
| | **Laboratory scale (twelve months)** | | **larged scale (six months)** |
| | **Ot.F.** | **Max.F.** | **Max.F.** |
| **AHD (nm)** | 339.9 ± 5,2 | 345.9 ± 8,3 | 345.4 ± 2,1 |
| **PI** | 0.20 ± 0,06 | 0.21 ± 0,01 | 0.15 ± 0,02 |
| **ZP (mV)** | -29.1 ± 3,1 | -42.7 ± 6,2 | -39.9 ± 0,2 |

| | | | |
|---|---|---|---|
| Ot.F.: optimized formulation; Max.F.: formulation with maximum concentration of borage oil; AHD: average hydrodynamic diameter; PI: polydispersity index; ZP: zeta potential. | | | |

### EXAMPLE 9 - Encapsulation of borage oil formulation free of polyol and with cyclosporine in nanoemulsion by the DPE method (Example 9)

The borage oil nanoemulsion, in addition to its intrinsic properties mentioned above, constitutes a platform for encapsulation of drugs with low solubility in water. Nanoemulsions containing cyclosporine were prepared by the DPE method, using borage oil as the lipid phase. Initially, 2 formulations were prepared using the DPE method, 1 of which contained 10% (m/m) of cyclosporine (100 mg/mL) and the other containing 1% (m/m) of cyclosporine (10 times less, 10 mg/mL). The concentrations mentioned were selected, since 100 mg/mL is the concentration used in veterinary medicines for the treatment of chronic manifestations of atopic dermatitis in dogs and symptomatic treatment of chronic allergic dermatitis in cats. The justification for reducing the concentration was based on the intrinsic property of nanoparticles, which have a greater area/surface to volume ratio, requiring a lower drug concentration to achieve the desired therapeutic effect.

In the prepared formulations, cyclosporine was solubilized in borage oil, at room temperature, under magnetic stirring (300 rpm), for approximately 1 hour. Subsequently, the **formulations were prepared according to the steps described in the item "Nanoemulsion Preparation".**

In formulations containing 10% (w/w) cyclosporine, cloudiness was observed in the oil after the drug was dissolved. The polyol-free formulations were prepared and showed good appearance after preparation, with AHD of 285.3 nm, PI of 0.44 and ZP of -26.6 mV. However, after 24 hours, sedimentation was observed at the bottom of the vial, which may be associated with cyclosporine that might not have been fully solubilized in the borage oil.

Subsequently, another polyol-free formulation was prepared, containing ten times less cyclosporine, that is, 1% (w/w). During the drug solubilization process in borage oil, it was observed that the oil remained completely translucent, indicating that cyclosporine was completely solubilized. The polyol-free formulations were prepared and showed AHD of 223.5 nm, PI of 0.16 and ZP of -27.0 mV.

It should be understood that the present description does not limit the application to the details described herein and that the invention is capable of other embodiments and of being practiced or executed in a variety of ways, within the scope of the claims. Although specific terms have been used, such terms should be interpreted in a generic and descriptive sense, and not for the purpose of limiting the scope of the invention.

### Bibliographic References

ANTON, N.; BENOIT, J.-P.; SAULNIER, P. Design and production of nanoparticles formulated from nano-emulsion templates-A review. Journal of Controlled Release, v. 128, n. 3, p. 185-199, jun. 2008.

BACCOUCH, R. et al. The impact of lipid polyunsaturation on the physical and mechanical properties of lipid membranes. Biochimica et Biophysica Acta (BBA) - Biomembranes, v. 1865, n. 2, p. 184084, fev. 2023.

BALIĆ, A. et al. Omega-3 Versus Omega-6 Polyunsaturated Fatty Acids in the Prevention and Treatment of Inflammatory Skin Diseases. International Journal of Molecular Sciences, v. 21, n. 3, p. 741, 23 jan. 2020.

DENNIS, E. A.; NORRIS, P. C. Eicosanoid storm in infection and inflammation. Nature Reviews Immunology, v. 15, n. 8, p. 511-523, 3 ago. 2015.

D'ERRICO, G.; CICCARELLI, D.; ORTONA, O. Effect of glycerol on micelle formation by ionic and nonionic surfactants at 25 °C. Journal of Colloid and Interface Science, v. 286, n. 2, p. 747-754, jun. 2005.

DODSON, C. D.; STERMITZ, F. R. Pyrrolizidine Alkaloids from Borage (Borago officinalis) Seeds and Flowers. Journal of Natural Products, v. 49, n. 4, p. 727-728, 1 jul. 1986.

ENDOO, M.; SAGITANI, H. Preparation of Triglyceride O/W Emulsions by D Phase Emulsification. Journal of Japan Oil Chemists' Society, v. 40, n. 2, p. 133-139, 1991.

FAGIONATO MASIERO, J. et al. Vegetable oils in pharmaceutical and cosmetic lipid-based nanocarriers preparations. Industrial Crops and Products, v. 170, p. 113838, out. 2021.

Guidance for Industry Considering Whether an FDA- Regulated Product Involves the Application of Nanotechnology. Biotechnology Law Report, v. 30, n. 5, p. 613-616, set. 2011.

GUTIERREZ, J. M. et al. Nano-emulsions: New applications and optimization of their preparation. Current Opinion in Colloid & Interface Science, v. 13, n. 4, p. 245-251, ago. 2008.

K., S.; KUMAR, A. Nanoemulsions: Techniques for the preparation and the recent advances in their food applications. Innovative Food Science & Emerging Technologies, v. 76, p. 102914, mar. 2022.

KANOST, M. R. Hemolymph. Em: Encyclopedia of Insects. [s.l.] Elsevier, 2009. p. 446-449.

KOROLEVA, M.; NAGOVITSINA, T.; YURTOV, E. Nanoemulsions stabilized by non-ionic surfactants: stability and degradation mechanisms. Physical Chemistry Chemical Physics, v. 20, n. 15, p. 10369-10377, 2018.

KUNIEDA, H. et al. Highly Concentrated Cubic- Phase Emulsions: Basic Study on D-Phase Emulsification using Isotropic Gels. Journal of Oleo Science, v. 50, n. 8, p. 633- 639, 2001.

LOH, J. M.; ADENWALLA, N.; WILES, S.; PROFT, T. Galleria mellonella larvae as an infection model for group A streptococcus. Virulence, v. 4, n. 5, p. 419-428, 2013.

MASIERO, Jéssica Fagionato et al. Unveiling the optimal path: High-pressure homogenization and D-phase emulsification methods for borage oil nanoemulsion design. Industrial Crops and Products, v. 208, p. 117853, 2024.

MIERENDORFF, H. -J. Bestimmung von Pyrrolizidinalkaloide durch Dünnschichtchromatographie in Samenölen von Borago off. L. Lipid / Fett, v. 97, n. 1, p. 33-37, 24 jan. 1995.

MONTGOMERY, D. C. Design and Analysis of Experiments. 10. ed. [s.l.] Wiley, 2019.

MURAKAMI, A. et al. Effects of Sugars on the D Phase Emulsification of Triglyceride Using Polyoxyethylene Sorbitan Fatty Acid Ester. Journal of Oleo Science, v. 54, n. 12, p. 633-639, 2005.

PARVAIS, O. et al. TLC detection of pyrrolizidine alkaloids in oil extracted from the seeds of Borago officinalis. JPC. Journal of planar chromatography, modern TLC, v. 7, n. 1, p. 80-82, 1994.

ROCCHIO, J. et al. A solvent-free lecithin-Tween 80 system for oil dispersion. Colloids and Surfaces A: Physicochemical and Engineering Aspects, v. 533, p. 218-223, nov. 2017.

SAGITANI, H. Formation of Fine Emuisions by Surface Chemical Methods. Journal of Japan Oil Chemists' Society, v. 35, n. 3, p. 198-202, 1986.

SAGITANI, H. FORMATION OF O/W EMULSIONS BY SURFACTANT PHASE EMULSIFICATION AND THE SOLUTION BEHAVIOR OF NONIONIC SURFACTANT SYSTEM IN THE EMULSIFICATION PROCESS.

Journal of Dispersion Science and Technology, v. 9, n. 2, p. 115-129, jan. 1988.

SAGITANI, H.; IKEDA, Y.; OGO, Y. Effect of Polyols on the Association State of Nonionic Surfactants in Aqueous Solutions. Journal of Japan Oil Chemists' Society, v. 33, n. 3, p. 156-161, 1984.

SAGITANI, H.; NABETA, K.; NAGAI, M. A New Preparing Method for Fine O/W Emulsions by D Phase Emulsification and Their Application to Cosmetic Industry. Journal of Japan Oil Chemists' Society, v. 40, n. 11, p. 988-994, 1991.

SOLANS, C.; SOLÉ, I. Nano-emulsions: Formation by low-energy methods. Current Opinion in Colloid & Interface Science, v. 17, n. 5, p. 246-254, out. 2012.

TAYLOR, P. Ostwald ripening in emulsions. Advances in Colloid and Interface Science, v. 75, n. 2, p. 107- 163, abr. 1998.

VACILLOTTO, G. et al. A rapid and highly specific method to evaluate the presence of pyrrolizidine alkaloids in Borago officinalis seed oil. Journal of Mass Spectrometry, v. 48, n. 10, p. 1078-1082, out. 2013.

YU, X.-L.; HE, Y. Application of Box-Behnken designs in parameters optimization of differential pulse anodic stripping voltammetry for lead(II) determination in two electrolytes. Scientific Reports, v. 7, n. 1, p. 2789, 5 jun.

### 2017.

YUKUYAMA, M. N. et al. Olive oil nanoemulsion preparation using high-pressure homogenization and d-phase emulsification - A design space approach. Journal of Drug Delivery Science and Technology, v. 49, p. 622-631, fev. 2019.

YUKUYAMA, M. N.; GHISLENI, D. D. M.; PINTO, T. J. A. Nanoemulsion: process selection and application in cosmetics - a review. p. 13-24, 2016.

## Claims

1. PROCESS OF PRODUCING VEGETABLE OIL NANOEMULSION **characterized in that** it is free of polyol using phase D emulsification (DPE), the process comprising the following steps:
a) homogenizing the aqueous phase comprising 0.5 to 1.5% initial water and 1 to 5% non-ionic surfactant under agitation of 50 to 500 rpm, preferably 300 rpm, at a temperature of 10 to 50°C, preferably 25°C;
b) adding 5 to 50% of the volume of vegetable oil to the aqueous phase with controlled speed under agitation of 50 to 500 rpm, preferably 300 rpm;
c) adding the remaining volume of vegetable oil, and maintaining agitation at 50 to 500 rpm, preferably 300 rpm for 10 to 30 minutes, preferably 20 minutes;
d) adding the remaining water to the total volume of the preparation; and
e) maintaining constant agitation of the vegetable oil nanoemulsion at 50 to 500 rpm, preferably 300 rpm for 10 to 30 minutes, preferably 20 minutes.

2. PROCESS, according to claim 1, **characterized in that** the nonionic surfactant is selected from the group: polysorbate 20, polysorbate 40, polysorbate 60, polysorbate 80, sorbitan monolaurate, sorbitan monopalmitate, sorbitan monoleate, sorbitan trioleate, lecithin, glyceryl stearate, glyceryl laurate, glyceryl oleate, glyceryl cetearate, caprylyl/capryl glucoside, decyl glucoside, lauryl polyglucoside, glyceryl isostearate, cetyl alcohol ethoxylate, octoxynol-9, nonoxynol-9, poloxamer 188, poloxamer 407, poloxamine 904, polyethylene glycol 400, polyethylene glycol
600, polyethylene glycol 1000, polyethylene glycol 3350, PEG-8 dimethicone, PEG-12 dimethicone, lauryl methacrylate, cholesterol ethoxylate, methylglucamine, polyethylene glycol distearate, methylglucoside ester, methylglucoside phosphate, polyglyceryl-4 ester, polyglyceryl laurate-10, glyceryl ricinoleate.

3. PROCESS, according to claim 2, **characterized in that** the non-ionic surfactant is preferably polysorbate 80.

4. PROCESS, according to claim 1, **characterized in that** the vegetable oil can be selected from the group of: avocado oil (*Persea americana*), sweet almond oil (*Prunus dulcis*), andiroba oil (*Carapa guianensis*), argan oil (*Argania spinosa*), hazelnut oil (*Corylus avellana*), babassu oil (*Orbignya phalerata*), baobab oil (*Adansonia digitata*), buriti oil (*Mauritia flexuosa*), camellia oil (*Camellia oleifera*), canola oil (*Brassica napus*), Brazil nut oil (*Bertholletia excelsa*), Maranhão nut oil (*Sterculia striata*), safflower oil (*Carthamus tinctorius*), chia oil (*Salvia hispanica*), coconut oil (*Cocos nucifera*), copaiba oil (*Copaifera spp*.), crambe oil (*Crambe abyssinica*), apricot oil (*Prunus armeniaca*), prickly pear oil (*Opuntia ficus-indica*), rice bran oil (*Oryza sativa*), raspberry oil (*Rubus idaeus*), sunflower oil (*Helianthus annuus*), sesame oil (*Sesamum indicum*), guarana oil (*Paullinia cupana*), shea oil (*Vitellaria paradoxa*), lavender oil (*Lavandula angustifolia*), linseed oil (*Linum usitatissimum*), macadamia oil (*Macadamia integrifolia*), castor oil (*Ricinus communis*), passion fruit oil (*Passiflora edulis*), marula oil (*Sclerocarya birrea*), tea tree oil (*Melaleuca alternifolia*), corn oil (*Zea mays*), moringa oil (*Moringa oleifera*), mustard oil (*Brassica juncea*), neem oil (*Azadirachta indica*), pecan oil (*Carya illinoinensis*), olive oil (*Olea europaea*), pequi oil (*Caryocar brasiliense*), patauá oil (*Oenocarpus bataua*), pracaxi oil (*Pentaclethra macroloba*), rosehip oil (*Rosa rubiginosa*), pumpkin seed oil (*Cucurbita pepo*), cottonseed oil (*Gossypium hirsutum*), grape seed oil (*Vitis vinifera*), tamanu oil (*Calophyllum inophyllum*), tucuma oil (*Astrocaryum vulgare*), cashew oil (*Anacardium occidentale*), cupuaçu oil (*Theobroma grandiflorum*), cocoa oil (*Theobroma cacao*), emburana oil (*Amburana cearensis*), peanut oil (*Arachis hypogaea*), black currant oil (*Ribes nigrum*), seaweed oil (*Schizochytrium spp*.), krill oil (*Euphausia superba*), borage oil (*Borago officinalis*), fennel oil (*Foeniculum vulgare*), rosemary oil (*Rosmarinus officinalis*), milk thistle oil (*Silybum marianum*), prickly pear oil (*Opuntia ficus-indica*), annatto oil (*Bixa orellana*), chamomile oil (*Matricaria recutita*), peppermint oil (*Mentha piperita*), ginger oil (*Zingiber officinale*).

5. PROCESS, according to claim 4, **characterized in that** the vegetable oil is preferably borage oil.

6. PROCESS, according to claim 1, **characterized in that** the phase D emulsification (FDE) process uses low energy.

7. VEGETABLE OIL NANOEMULSION, **characterized in that** the concentration of vegetable oil is higher than 5 to 50% w/w, preferably 50% w/w, the concentration of the non-ionic surfactant is at most 2 to 5% w/w, preferably 4.0% w/w, and **in that** the non-ionic surfactant is selected from the group consisting of: polysorbate 20, polysorbate 40, polysorbate 60, polysorbate 80, sorbitan monolaurate, sorbitan monopalmitate, sorbitan monooleate, sorbitan trioleate, lecithin, glyceryl stearate, glyceryl laurate, glyceryl oleate, glyceryl ceteareth, caprylyl/capryl glucoside, decyl glucoside, lauryl polyglucoside, glyceryl isostearate, ethoxylated cetyl alcohol, octoxynol-9, nonoxynol-9, poloxamer 188, poloxamer 407, poloxamine 904, polyethylene glycol 400, polyethylene glycol 600, polyethylene glycol 1000, polyethylene glycol 3350, PEG-8 dimethicone, PEG-12 dimethicone, lauryl methacrylate, cholesterol ethoxylate, methylglucamine, polyethylene glycol distearate, methyl glucose ester, methyl glucose phosphate, polyglyceryl-4 ester, polyglyceryl-10 laurate, glyceryl ricinoleate, preferably polysorbate 80.

8. VEGETABLE OIL NANOEMULSION, according to claim 7, **characterized in that** the vegetable oil can be selected from the group of: avocado oil (*Persea americana*), sweet almond oil (*Prunus dulcis),* andiroba oil (*Carapa guianensis*), argan oil (*Argania spinosa*), hazelnut oil (*Corylus avellana),* babassu oil (*Orbignya phalerata*), baobab oil (*Adansonia digitata*), buriti oil (*Mauritia flexuosa*), camellia oil (*Camellia oleifera*), canola oil (*Brassica napus*), Brazil nut oil (*Bertholletia excelsa*), Maranhão nut oil (*Sterculia striata*), safflower oil (*Carthamus tinctorius*), chia oil (*Salvia hispanica*), coconut oil (*Cocos nucifera*), copaiba oil (*Copaifera spp*.), crambe oil (*Crambe abyssinica*), apricot oil (*Prunus armeniaca*), prickly pear oil (*Opuntia ficus-indica*), rice bran oil (*Oryza sativa*), raspberry oil (*Rubus idaeus*), sunflower oil (*Helianthus annuus*), sesame oil (*Sesamum indicum*)*,* guarana oil (*Paullinia cupana*), shea oil (*Vitellaria paradoxa*), lavender oil (*Lavandula angustifolia*), linseed oil (*Linum usitatissimum*), macadamia oil (*Macadamia integrifolia*), castor oil (*Ricinus communis*), passion fruit oil (*Passiflora edulis*), marula oil (*Sclerocarya birrea),* tea tree oil (*Melaleuca alternifolia*), corn oil (*Zea mays*), moringa oil (*Moringa oleifera*), mustard oil (*Brassica juncea*), neem oil (*Azadirachta indica*), pecan oil (*Carya illinoinensis*), olive oil (*Olea europaea*), pequi oil (*Caryocar brasiliense*), patauá oil (*Oenocarpus bataua*), pracaxi oil (*Pentaclethra macroloba*), rosehip oil (*Rosa rubiginosa*), pumpkin seed oil (*Cucurbita pepo*), cottonseed oil (*Gossypium hirsutum*), grape seed oil (*Vitis vinifera*), tamanu oil (*Calophyllum inophyllum*), tucuma oil (*Astrocaryum vulgare*), cashew oil (*Anacardium occidentale*), cupuagu oil (*Theobroma grandiflorum*), cocoa oil (*Theobroma cacao*), emburana oil (*Amburana cearensis*), peanut oil (*Arachis hypogaea*), black currant oil (*Ribes nigrum*), seaweed oil (*Schizochytrium spp*.), krill oil (*Euphausia superba*), borage oil (*Borago officinalis*), fennel oil (*Foeniculum vulgare*), rosemary oil (*Rosmarinus officinalis*), milk thistle oil (*Silybum marianum*), prickly pear oil (*Opuntia ficus-indica*), annatto oil (*Bixa orellana*), chamomile oil (*Matricaria recutita*), peppermint oil (*Mentha piperita*), ginger oil (*Zingiber officinale*).

9. VEGETABLE OIL NANOEMULSION, according to claim 8, **characterized in that** the vegetable oil is borage oil.

10. VEGETABLE OIL NANOEMULSION according to any one of claims 7 to 9, **characterized in that** it comprises an average hydrodynamic diameter of less than 500 nm, preferably 350 nm, the polydispersity index is between 0.05 and 0.300 and the zeta potential is between -48.9 and -29.1 mV.

11. VEGETABLE OIL NANOEMULSION, according to any one of claims 7 to 10, **characterized in that** it is a platform for encapsulation of drugs with low water solubility selected from the group of immunosuppressants, anti-inflammatories, antihistamines, antibiotics.

12. VEGETABLE OIL NANOEMULSION, according to claim 11, **characterized in that** the immunosuppressants are selected from the group of: azathioprine, cyclosporine, tacrolimus, sirolimus (rapamycin), everolimus, temsirolimus, zotarolimus, leflunomide, mycophenolate mofetil, mycophenolate sodium, gusperimus, pimecrolimus, fingolimod, baricitinib, ruxolitinib, tofacitinib, abatacept, belatacept, voclosporin, laquinimod; steroidal anti-inflammatory drugs are selected from the group of: dexamethasone, betamethasone, budesonide, clobetasol, diflorasone, fluocinolone acetonide, fluocortolone, fluticasone, halcinonide, hydrocortisone, methylprednisolone, mometasone, paramethasone, prednisolone, prednisone, triamcinolone, desoximethasone, cyclohexanolone, beclomethasone, alclomethasone; non-steroidal anti-inflammatory drugs are selected from the group of: aceclofenac, mefenamic acid, niflumic acid, tiaprofenic acid, amtolmetin guacil, benoxaprofen, bromfenac, carprofen, celecoxib, ketoprofen, clonixin, dexibuprofen, dexketoprofen, diclofenac, diflunisal, etodolac, etofenamate, phenylbutazone, flurbiprofen, ibuprofen, indomethacin, ketorolac, lornoxicam, lumiracoxib, meclofenamate, meloxicam, nabumetone, naproxen, nimesulide, oxaprozin, parecoxib, piroxicam, proglumetacin, rofecoxib, sulindac, tenoxicam, valdecoxib, tolmetin, azapropazone, feprazone; antihistamines are selected from the group of: astemizole, bilastine, ebastine, loratadine, desloratadine, mizolastine, terfenadine, clemastine, chlorpheniramine, dexchlorpheniramine, brompheniramine, diphenhydramine, promethazine, doxylamine, cyproheptadine, meclizine, hydroxyzine, triprolidine, tripelennamine, dimetindene, oxatomide, azelastine, buclizine, chlorcyclizine, ketotifen, alimemazine, antazoline, trimeprazine, pheniramine, dexbrompheniramine; and antibiotics are selected from the group of: azithromycin, clarithromycin, erythromycin base, telithromycin, clindamycin, lincomycin, rifampicin, rifabutin, rifapentine, isoniazid, ethionamide, para-aminosalicylic acid, dapsone, chloramphenicol, tiamulin, valnemulin, gramicidin, bacitracin, polymyxin B, colistin, griseofulvin, sodium fusidate, streptomycin, amikacin, capreomycin, spectinomycin, cephradine, cefaclor, cefixime, cefdinir, cefpodoxime, ceftibutene, minocycline, doxycycline, tetracycline, tigecycline, ciprofloxacin, levofloxacin, moxifloxacin, norfloxacin, ofloxacin, trovafloxacin, besifloxacin, gatifloxacin, gemifloxacin, sitafloxacin, prulifloxacin, finafloxacin, nadifloxacin, enoxacin.

13. VEGETABLE OIL NANOEMULSION, according to claim 11, **characterized in that** the immunosuppressant is cyclosporine.

14. PHARMACEUTICAL COMPOSITION **characterized in that** it comprises 1% (w/w) of cyclosporine solubilized in the polyol-free borage oil nanoemulsion as defined in any one of claims 7 to 13.

15. COMPOSITION, according to claim 14, **characterized in that** it comprises a AHD of 223.5 nm, PI of 0.16 and ZP of -27.0 mV.

16. USE OF THE PHARMACEUTICAL COMPOSITION as defined in claims 14 or 15, **characterized in that** it is for the manufacture of a medicine for the treatment of atopy, hypercholesterolemia, premenstrual syndrome, insulin resistance, in supporting wound healing, diabetic neuropathy, metabolic syndrome, preferably atopic dermatitis in mammals.
USE, according to claim 16, **characterized in that** mammals are selected from humans and animals selected from the group consisting of dogs, cats, horses, cattle, sheep, goats, pigs, rabbits, hamsters, guinea pigs, rats, preferably dogs and cats.
